# EUROPÄISCHE PATENTSCHRIFT

(11) **EP 2 401 953 B1**
(45) Veröffentlichungstag und Bekanntmachung des Hinweises auf die Patenterteilung: **28.10.2020**
(21) Anmeldenummer: 11005115.8
(22) Anmeldetag: 24.06.2011
(51) Int. Cl.: A61B 1/00, G02B 23/24, G03B 17/48

(54) **Kupplung zum lösbaren Verbinden eines Okulars eines Endoskops mit einer Kamera**
Coupling device for a detachable connection between the ocular of an endoscope and a camera
Dispositif de couplage pour une connexion amovible entre l'oculaire d'un endoscope et une caméra

(30) Priorität: 29.06.2010 DE 102010025556
(43) Veröffentlichungstag der Anmeldung: 04.01.2012
(73) Patentinhaber: Richard Wolf GmbH, 75438 Knittlingen (DE)
(72) Erfinder: Faber, Fredy, Dipl.- Ing., 75438 Knittlingen (DE); Lampert, Alexander, Dipl.- Ing., 75248 Ölbronn-Dürrn (DE); Michels, Andreas, Dipl.- Ing., 75045 Walzbachtal (DE)
(74) Vertreter: Patentanwälte Vollmann Hemmer Lindfeld Partnerschaft mbB

(56) Entgegenhaltungen:
- DE-A1- 2 757 358
- DE-A1- 3 014 116
- DE-U1-202008 000 449
- JP-A- 10 243 917

## Beschreibung

Die Erfindung betrifft eine Kupplungsvorrichtung zum lösbaren Verbinden eines Okulars einer Endoskop-Optik mit einem Kameraobjektiv mit den im Oberbegriff des Anspruchs 1 angegebenen Merkmalen.

Kupplungsvorrichtungen dieser Art dienen dazu, ein ansonsten für die Betrachtung mit dem Auge dienendes Okular eines endoskopischen Instruments an das Objektiv einer Kamera anzuschließen. Eine solche Kupplungsvorrichtung ist aus DE 69 26 837 U bekannt. Bei dieser Vorrichtung ist ein Ringelement vorgesehen, das in das distale Ende eines Objektivgehäuses einschraubbar ist. In das Ringelement greift ein Innenring ein, der eine Aufnahme für den konusstumpfförmig ausgebildeten proximalen Endabschnitt eines Okulars bildet. Über den Umfang verteilt, weist der Innenring mehrere radiale Durchbohrungen auf, die jeweils zur Aufnahme einer Sperrkugel dienen. Der Innenring ist von einem drehbaren ringförmigen Griffteil umgeben. Dieses Griffteil ist an seinem Innenumfang korrespondierend zu den Durchbohrungen des Innenrings mit länglichen Ausnehmungen versehen, die in Umfangsrichtung des Griffteils kontinuierlich tiefer werden. Das Griffteil ist entgegen seiner Drehrichtung gegenüber dem Ringelement federvorgespannt. Bei einem nicht in dem Innenring eingeführten Okular befinden sich die Sperrkugeln immer in ihrer, den konischen Abschnitt des Okulars ansonsten festlegenden Stellung. Um das Okular in den Innenring einführen zu können, muss das Griffteil gegen seine Federvorspannung so gedreht werden, dass die Sperrkugeln in die an dem Griffteil ausgebildeten Ausnehmungen an deren tiefster Stelle eingreifen können. Durch Loslassen des Griffteils wird dieses durch Federentspannung bewegt und die Sperrkugeln hierdurch entsprechend der abnehmenden Tiefe der Ausnehmungen des Griffteils in den Durchbohrungen des Innenrings nach innen gedrückt, wo sie dann an dem Innenumfang des Innenrings herausragen und den konischen Teil des Okulars umgreifen und auf diese Weise festlegen. Zum Lösen des Okulars in der Kupplungsvorrichtung ist das Griffteil wieder entgegen seiner Federvorspannung zu drehen, damit sich die Sperrkugeln wieder nach außen, also von dem Okular weg, in die an dem Griffteil ausgebildeten Ausnehmungen bewegen können.

In DE 27 57 358 A1 wird eine Kupplungsvorrichtung offenbart, welche selbsttätig ein in die Aufnahme der Vorrichtung eingeführtes Okular verriegelt. Zum Einsetzen des Okulars in die Kupplungsvorrichtung müssen die zur Befestigung vorgesehenen Kipphebel entgegen der Kraft der Federn gedreht werden. Dies erfolgt durch Betätigung eines Knopfes, der an einem Betätigungsring umfänglich der Kupplungsvorrichtung vorgesehen ist. Es muss also gleichzeitig dieser Knopf in Umfangsrichtung gedrückt werden und das Okular in die Aufnahme eingeführt werden, wonach dann erst selbsttätig eine Befestigung bzw. Verriegelung des Okulars in der Aufnahme erfolgt. In gleicher Weise ist eine solche Betätigung des Knopfes zum Entriegeln und Lösen der Befestigung erforderlich. Zum Befestigen des Okulars in der Aufnahme sind somit zwei Hände erforderlich, eine zur Betätigung des Knopfes und die andere zum Einführen des Okulars, was nachteilig ist.

In DE 30 14 116 A1 ist eine Kupplungsvorrichtung zum Befestigen eines Kameraobjektivs an einem Okular eines endoskopischen Instrumentes beschrieben, die einen Aufnahmeraum für das Okular aufweist, in dem das Okular mittels schwenkbarer Hebel festgelegt wird. Die Hebel werden von einem drehbaren, federvorgespannten Sicherungsring in der das Okular festlegenden Stellung gehalten. Der Sicherungsring ist von einer Stellung, in der er die Hebel in einer das Okular festlegenden Stellung hält, in eine Stellung drehbar, in der die Hebel das Okular freigeben. Vor dem Einführen des Okulars in den Aufnahmeraum der Kupplungsvorrichtung muss der Sicherungsring in die Stellung gedreht werden, in der die Hebel das Okular freigeben und so lange in dieser Stellung gehalten werden, bis sich das Okular in dem Aufnahmeraum der Kupplungsvorrichtung befindet.

JP 10-243917 ist eine weitere Kupplungsvorrichtung zum Befestigen eines Kameraobjektivs an einem Okular eines endoskopischen Instrumentes zu entnehmen, die einen Aufnahmeraum für das Okular aufweist, in dem das Okular mittels schwenkbarer Hebel festgelegt wird.

Aus DE 20 2008 000 449 U1 ist eine Kupplungsvorrichtung zum Befestigen eines Kameraobjektivs an einem Okular eines endoskopischen Instrumentes bekannt, bei der das Okular, nachdem es in die Kupplungsvorrichtung eingeführt worden ist, von hinter dem Okulartrichter einrastenden Verriegelungselementen gehalten wird. Zum Trennen der Verbindung zwischen dem Okular und der Kupplungsvorrichtung ist am Außenumfang der Kupplungsvorrichtung ein Auslöser angeordnet.

Vor diesem Hintergrund liegt der Erfindung die Aufgabe zugrunde, eine Kupplungsvorrichtung zum lösbaren Verbinden eines Okulars einer Endoskop-Optik mit einem Kameraobjektiv zu schaffen, die einfacher als die bislang bekannten Kupplungsvorrichtungen dieser Art zu bedienen ist.

Gelöst wird diese Aufgabe durch eine Kupplungsvorrichtung mit den in Anspruch 1 angegebenen Merkmalen. Vorteilhafte Weiterbildungen dieser Kupplungsvorrichtung ergeben sich aus den Unteransprüchen, der nachfolgenden Beschreibung sowie der Zeichnung. Hierbei können gemäß der Erfindung die in den Unteransprüchen angegebenen Merkmale jeweils für sich aber auch in technologisch sinnvoller Kombination die erfindungsgemäße Lösung gemäß Anspruch 1 weiter ausgestalten.

Die erfindungsgemäße Kupplungsvorrichtung zum lösbaren Verbinden eines Okulars einer Endoskop-Optik mit einem Kameraobjektiv weist eine Aufnahme für das Okular auf. Dabei dient die Aufnahme zur Aufnahme eines sich konusstumpfförmig erweiternden proximalen Endabschnitts des Okulars, der üblicherweise auch als Okulartrichter bezeichnet wird. Des Weiteren weist die erfindungsgemäße Kupplungsvorrichtung Befestigungsmittel zur Befestigung des Okulars in der Aufnahme und ein außenseitig der Kupplungsvorrichtung angeordnetes, manuell betätigbares Griffteil zum Lösen der Befestigung auf.

Gemäß der Erfindung sind die Befestigungsmittel durch das Okular in einer Art und Weise gesteuert, dass beim Einführen des Okulars in die Aufnahme eine selbsttätige Befestigung des Okulars in der Aufnahme erfolgt. D.h., beim Einführen des Okulars in die Aufnahme der erfindungsgemäßen Kupplungsvorrichtung, ist es anders als beim Stand der Technik vorteilhafterweise nicht mehr erforderlich, zuvor eine Verriegelung zu lösen. Die Befestigungsmittel werden beispielsweise durch einen direkten Kontakt des Okulars mit geeigneten Steuermitteln, die mit den Befestigungsmitteln wirkungsverbunden sind, derart angesteuert, dass sie das Okular in der Aufnahme festlegen, ohne dass hierzu eine weitere Betätigung an der Kupplungsvorrichtung erforderlich ist.

Das Griffteil ist bei der erfindungsgemäßen Kupplungsvorrichtung nur zum Lösen der Befestigung manuell betätigbar, d.h., es muss nur betätigt werden, wenn das in der Aufnahme der Kupplungsvorrichtung befestigte Okular von der Kupplungsvorrichtung getrennt werden soll. Zweckmäßigerweise ist das Griffteil an einem solchen Bereich der Außenseite der Kupplungsvorrichtung angeordnet, an dem es für den Benutzer der Kupplungsvorrichtung besonders gut zugänglich ist.

Zum Lösen des Okulars ist das Griffteil gegen Federkraft verschiebbar. Demzufolge ist das Griffteil als ein Schieber ausgebildet. Die Verschieberichtung des Griffteils zum Lösen des Okulars stimmt bei der erfindungsgemäßen Kupplungsvorrichtung mit der Einführrichtung des Okulars in die Aufnahme überein. Eine solche Bewegung des Griffteils wird in der Regel spontan bzw. intuitiv in Betracht gezogen werden, da diese Bewegung auch dann, wenn das Okular nicht in der Aufnahme festgelegt ist, zu einer Trennung des Okulars von der Kupplungsvorrichtung führen würde.

Die Ansteuerung der Befestigungsmittel durch das Okular erfolgt bei der erfindungsgemäßen Kupplungsvorrichtung über dafür vorgesehene Steuermittel. So kann in der Aufnahme vorteilhaft mindestens ein bevorzugt federvorgespanntes Steuerelement bewegbar angeordnet sein, das mit einem Befestigungselement zum Festlegen des Okulars in der Aufnahme bewegungsgekoppelt ist. Zweckmäßigerweise ist das Steuerelement in der Aufnahme derart angeordnet, dass es beim Einführen des Okulars in die Aufnahme von dem Okular kontaktiert und gegen seine Federvorspannung bewegt wird, wobei diese Bewegung auf das Befestigungselement in einer Weise übertragen wird, dass es zu einem Kraftschluss und/oder vorzugsweise zu einem Formschluss zwischen dem Befestigungselement und dem Okular kommt, wodurch das Okular in der Aufnahme festgelegt wird.

Zur Bewegungskopplung mit dem Befestigungselement kann das Steuerelement über Getriebemittel mit dem Befestigungselement verbunden sein. Ein einfacherer Aufbau der Kupplungsvorrichtung lässt sich dadurch realisieren, wenn, wie es bevorzugt vorgesehen ist, ein Bauteil sowohl das Steuerelement als auch das Befestigungselement bildet. Dementsprechend wird bei dieser Ausgestaltung die Einführbewegung des Okulars über einen Teil des Bauteils, der das Steuerelement bildet, direkt auf das Befestigungselement übertragen, derart, dass das Befestigungselement in eine Stellung bewegt wird, in der es das Okular festlegt.

Um die Festlegung des Okulars in der Aufnahme aufrechterhalten zu können, wird das Befestigungselement zweckmäßigerweise in dieser,

das Okular in der Aufnahme festlegenden, Stellung von dem Griffteil verriegelt Hierzu kann das Griffteil mit einer Feder gekoppelt sein und durch Entspannung dieser Feder, also selbsttätig ohne manuelle Betätigung, dann, wenn das Befestigungselement das Okular festlegt, in eine Stellung bewegt werden, in der es eine Bewegung des Befestigungselements verhindert.

Das Griffteil ist zum Lösen des Okulars in der Aufnahme der erfindungsgemäßen Kupplungsvorrichtung gegen Federkraft verschiebbar. Zweckmäßigerweise ist das Griffteil in der das Okular freigebenden Stellung, in der das Griffteil federvorgespannt ist, festzulegen. Zum Festlegen des Griffteils ist das Befestigungselement vorgesehen, das beim Lösen des Okulars vorteilhaft von der das Okular festhaltenden Stellung in eine das Okular freigebende Stellung bewegt wird und in letztgenannter Stellung das Griffteil gegen die wirkende Federkraft festhält.

Vorteilhaft weist die erfindungsgemäße Kupplungsvorrichtung einen abgewinkelten Hebel mit zwei Hebelarmen auf, wobei der Hebel um eine im Schnittbereich der beiden Hebelarme angeordnete Achse schwenkbar ist. Weiter ist bei dieser Ausgestaltung vorgesehen, dass ein erster Hebelarm zumindest teilweise in die Aufnahme hineinragt und ein im Wesentlichen senkrecht zu dem ersten Hebelarm ausgerichteter zweiter Hebelarm das Befestigungselement bildet. Bei dieser Ausgestaltung bildet der erste Hebelarm das Steuerelement und der zweite Hebelarm das Befestigungselement zum Festlegen des Okulars in der Aufnahme.

Bevorzugt ist der Hebel an der Aufnahme schwenkbeweglich gelagert. Zweckmäßigerweise ragt der erste, das Steuerelement bildende Hebelarm an einem von der Einführöffnung zum Einführen des Okulars abgewandten Ende der Aufnahme, das vorzugsweise einen Anschlag für das Okular bildet, in die Aufnahme hinein und kann dort von dem Okular zur Ansteuerung des das Betätigungselement bildenden zweiten Hebelarms kontaktiert werden. Hierbei kann der Hebel derart an der Aufnahme schwenkbeweglich gelagert sein, dass der das Befestigungselement bildende zweite Hebelarm zum Befestigen des Okulars durch eine an der Aufnahme ausgebildete Öffnung in das Innere der Aufnahme hineingeschwenkt werden kann und zum Freigeben des Okulars von diesem radial nach außen aus der Aufnahme herausgeschwenkt werden kann.

Bevorzugt ist der Hebel mittels eines Federbügels vorgespannt. Dieser Federbügel, bei dem es sich um ein Biegefederelement handelt, ist vorteilhaft derart angeordnet, dass das in die Aufnahme eingeführte Okular gegen dessen Federkraft im Wesentlichen senkrecht zu dessen Längsausdehnung wirkt. Nach dem Lösen des Okulars durch Betätigen des Griffteils bewirkt der Federbügel durch seine Entspannung, dass der Hebel zurück in eine Stellung bewegt wird, im der der Hebelarm, der das Steuerelement bildet, zumindest teilweise in die Aufnahme hineinragt und der Hebel, der das Befestigungselement bildet, aus dem Inneren der Aufnahme herausbewegt wird und so nicht den Zugang eines in die Aufnahme einzuführenden Okulars behindert.

Zum Befestigen des Okulars in der Aufnahme kann an dem das Befestigungselement bildenden Hebelarm vorteilhafterweise ein Vorsprung ausgebildet sein, der den Okulartrichter des Okulars in einer Befestigungsstellung formschlüssig umgreift. Hierzu kann der Vorsprung im Wesentlichen senkrecht zu der Längsausdehnung des zweiten Hebelarms ausgerichtet sein. Bevorzugt kann der Vorsprung so ausgebildet sein, dass er flächig an dem sich konisch erweiternden Bereich des Okulartrichters zur Anlage kommt.

Neben diesem Vorsprung ist an dem das Befestigungselement bildenden Hebelarm an der von dem Vorsprung abgewandten Seite bevorzugt ein zweiter Vorsprung ausgebildet. Dieser zweite Vorsprung stützt sich vorteilhaft in der das Okular festlegenden Stellung des Befestigungselementes an einer dem Befestigungselement zugewandten Innenseite des Griffteils ab, wodurch das Befestigungselement in dieser Stellung verriegelt wird.

Weiter vorteilhaft kann der zweite Vorsprung des das Befestigungselement bildenden zweiten Hebelarms zum Eingriff in eine an dem Griffteil ausgebildete Ausnehmung vorgesehen sein. Diese Ausnehmung ist zweckmäßigerweise an dem der Bewegungsrichtung beim Lösen des Okulars entgegen gesetzten Ende des Griffteils ausgebildet. Wird das Griffteil zum Lösen des Okulars gegen Federkraft bewegt, kann der Vorsprung in die Ausnehmung eingreifen und das Griffteil wie ein Haken gegen die wirkende Federkraft festlegen.

In einer weiteren bevorzugten Weiterbildung der erfindungsgemäßen Kupplungsvorrichtung können an dem Anschlussteil drei vorzugsweise im Winkelabstand von 120° voneinander beabstandete Hebel angeordnet sein. Dies ist insofern vorteilhaft, als ein in der Aufnahme befindliches Okular mittels der Hebelarme des Hebels, die die Befestigungselemente des Okular bilden, ähnlich einer Dreipunkt-Führung in der Aufnahme derart zentriert werden können, dass die optische Achse des Okulars mit der optischen Achse des Kameraobjektivs übereinstimmt.

Nachfolgend ist die Erfindung anhand eines in der Zeichnung dargestellten Ausführungsbeispiels näher erläutert. In der Zeichnung zeigt:

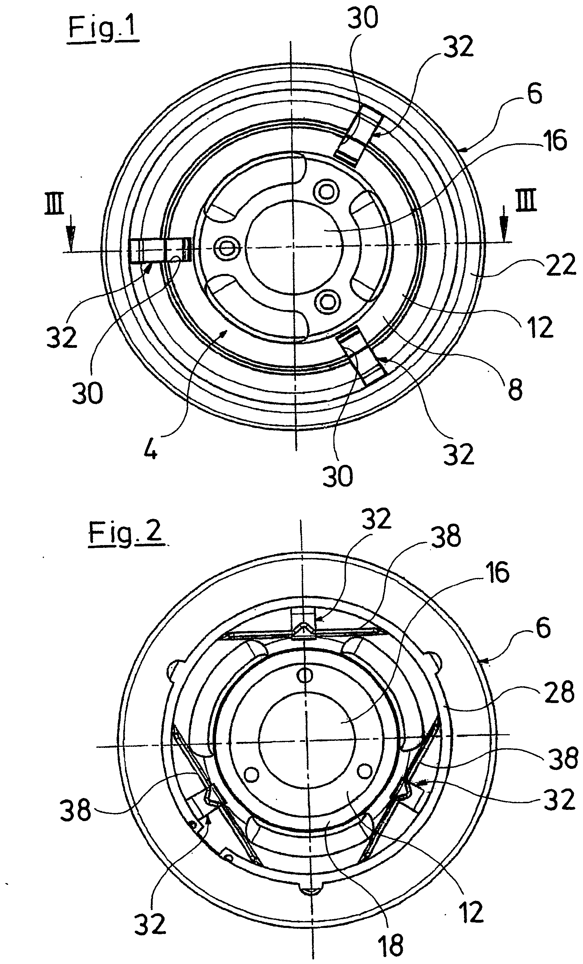

- Fig. 1: in schematischer Draufsicht in Einführrichtung eines Okulars eine Kupplungsvorrichtung,
- Fig. 2: die Kupplungsvorrichtung nach Fig. 1 in einer Draufsicht entgegen der Einführrichtung des Okulars,
- Fig. 3: die Kupplungsvorrichtung nach Fig. 1 in einer Schnittansicht entlang der Schnittlinie III - III in Fig. 1 und
- Fig. 4: die Kupplungsvorrichtung nach Fig. 1 in der Schnittansicht gemäß Fig. 3 mit eingeführtem Okular.

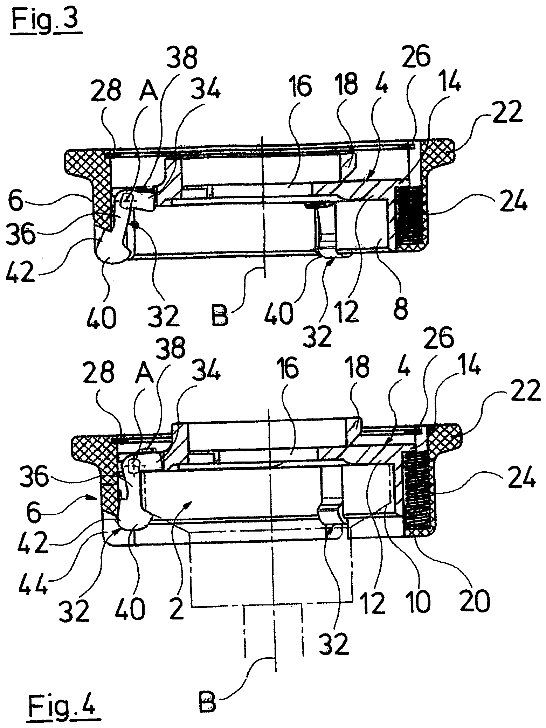

Zu den wesentlichen Bestandteilen der dargestellten Kupplungsvorrichtung zum lösbaren Verbinden eines Okulars 2 (in Fig. 4 strichpunktiert dargestellt) einer Endoskop-Optik mit einem in der Zeichnung nicht gezeigten Kameraobjektiv gehören eine Aufnahme 4 für das Okular 2 und ein Griffteil 6, mit dem eine Befestigung des Okulars 2 in der Aufnahme 4 gelöst werden kann.

Bei der Aufnahme 4 bildet ein hohlzylindrischer Abschnitt 8, dessen Innendurchmesser mit dem größten Außendurchmesser eines Okulartrichters 10 des Okulars 2 korrespondiert, einen Aufnahmeraum für den Okulartrichter 10. Zum Einführen des Okulars 2 ist der Abschnitt 8 an einem Ende offen ausgebildet. An dem anderen Ende des Abschnitts 8 sind ein radial nach innen ausgerichteter ringförmiger Kragen 12 und ein radial nach außen ausgerichteter ringförmiger Kragen 14 ausgebildet. Der Kragen 12 bildet eine Anlagefläche für ein in die Aufnahme 4 eingeführtes Okular 2. Eine von dem Kragen 12 gebildete Öffnung weist einen Durchmesser auf, der im Wesentlichen dem Durchmesser der Betrachtungslinse des Okulars 2 entspricht.

An der von der Einführöffnung für das Okular 2 abgewandten Seite des Kragens 12 schließt sich ein weiterer hohlzylindrischer Abschnitt 18 der Aufnahme 4 an. Dieser Abschnitt 18 ist koaxial zu dem Abschnitt 8 der Aufnahme 4 ausgerichtet. Der Abschnitt 18 dient zur Befestigung der Aufnahme 4 an einem Kameraobjektiv und bildet zusammen mit der an dem Kragen 12 des Abschnitts 8 ausgebildeten Öffnung einen optischen Kanal von dem Okular 2 zu dem Kameraobjektiv. Zur Befestigung an dem Kameraobjektiv weist der Abschnitt 18 an seiner äußeren Mantelfläche ein Gewinde auf, das mit einem an dem distalen Ende des Gehäuses des Kameraobjektivs ausgebildeten Innengewinde korrespondiert, so dass die Aufnahme 4 an dem Kameraobjektiv angeschraubt werden kann. Der Innendurchmesser des Abschnitts 18 entspricht im Wesentlichen dem Durchmesser einer distalen Linse des Kameraobjektivs.

Das Griffteil 6 bildet einen hohlzylindrischen Innenraum. In diesem Innenraum ist die Aufnahme 4 konzentrisch zu dem Griffteil 6 angeordnet, wobei die Aufnahme nahezu vollständig von dem Griffteil 6 aufgenommen wird. An dem der Einführöffnung für das Okular 2 der Aufnahme 4 zugewandten Ende des Griffteils 6 ist ein radial nach innen gerichteter ringförmiger Kragen 20 ausgebildet. An dem davon abgewandten Ende des Griffteils 6 weist das Griffteil 6 eine radial nach außen gerichtete flanschartige Erweiterung 22 auf, die eine Griffbegrenzung für eine das Griffteil 6 benutzende Person bildet.

Der Durchmesser der von dem Kragen 20 gebildeten Öffnung des Griffteils 6 korrespondiert mit dem Außendurchmesser des Abschnitts 8 der Aufnahme 4, wobei der an dem Abschnitt 8 ausgebildete Kragen 14 den an dem Griffteil 6 ausgebildeten Kragen 20 in radialer Richtung überlappt. Die Innenseite des Kragens 20 des Griffteils 6 dient als Auflager für dort gemäß der Darstellung nach Figur 3 und 4 aufrecht stehend angeordneten Schraubenfedern 24. An dem von dem Kragen 20 des Griffteils 6 abgewandten Enden der Schraubenfedern 24 stützt sich die Aufnahme 4 mit dem an dessen Abschnitt 8 ausgebildeten Kragen 14 ab. Im Bereich des von dem Kragen 20 abgewandten Endes des Griffteils 6 ist an dessen Innenumfang eine umlaufende Nut 26 ausgebildet.

Diese Nut 26 dient zur Aufnahme eines Sicherungsrings 28. Der Sicherungsring 28 überlappt in radialer Richtung den an dem Abschnitt 8 der Aufnahme 4 ausgebildeten Kragen 14 und verhindert ein Herausfallen der Aufnahme 4 aus dem Inneren des Griffteils 6.

An dem Abschnitt 8 sind über dessen Umfang verteilt jeweils in einem Winkelabstand von 120° drei Durchbrechungen 30 ausgebildet. Die Durchbrechungen 30 erstrecken sich jeweils durchgehend von einem radialen Außenbereich des Kragens 12 über die gesamte Länge der Mantelfläche des hohlzylindrischen Bereichs des Abschnitts 8 und bilden Zugangsöffnungen in das Innere des Abschnitts 8. Jede der Durchbrechungen 30 dient zur Aufnahme eines Hebels 32, der in der Durchbrechung 30 um eine Schwenkachse A im Bereich des Kragens 12 schwenkbeweglich gelagert ist.

Jeder der Hebel 32 ist abgewinkelt ausgebildet und weist zwei im Wesentlichen senkrecht zueinander ausgerichtete Hebelarme 34 und 36 auf, wobei die Hebelarme 34 im Wesentlichen in den Durchbrechungen 30 im Bereich des Kragens 12 und die Hebelarme 36 im Wesentlichen in den Durchbrechungen 30 im Bereich der Mantelfläche des Abschnitts 8 angeordnet sind. Wie Fig. 3 zu entnehmen ist greift der Hebelarm 34 jedes Hebels 32, wenn kein Okular 2 in den Abschnitt 8 der Aufnahme 4 eingeführt ist, teilweise in das Innere des Abschnitts 8 ein, während die Hebelarme 36 nicht in das Innere des Abschnitts 8 eingreifen. In dieser Stellung werden die Hebel 32 jeweils von einem Federbügel 38 gehalten. Hierzu sind die einzelnen Federbügel 38 derart an der Aufnahme 4 befestigt, dass sie an der von dem Abschnitt 8 der Aufnahme abgewandten Seite der Hebelarme 34 im Wesentlichen quer zu den Hebelarmen 34 ausgerichtet zur Anlage kommen und dort die Hebel 32 mit einer Druckkraft beaufschlagen.

Die Enden der Hebelarme 36 sind ballig erweitert ausgebildet, wobei sie einen ersten Vorsprung 40 bilden, der im Wesentlichen quer zur Längsausdehnung des jeweiligen Hebelarms 36 in Richtung des Inneren des Abschnitts 8 weist, und einen zweiten Vorsprung 42 bilden, der im Wesentlichen entgegengesetzt zum Vorsprung 40 ausgerichtet ist. Der Vorsprung 42 bildet jeweils einen kantigen Übergang zu dem übrigen Hebelarm 36.

An dem Griffteil 6 sind ausgehend von dem dort ausgebildeten Kragen 20 im Winkelabstand von 120° korrespondierend zu der Lage der Hebel 32 an der Aufnahme 4 Nuten 44 ausgebildet, die sich radial über die gesamte Breite des Kragens 20 und anschließend an der Mantelfläche des Griffteils 6 in dessen Längsrichtung erstrecken.

Die Funktionsweise der Kupplungsvorrichtung ist wie folgt:
Wie der Fig. 3 zu entnehmen ist, drücken die Federbügel 38 bei nicht in der Aufnahme 4 eingeführtem Okular 2 die Hebel 32 in eine Stellung, in der die Hebelarme 34 im Bereich des Kragens 12 des Abschnitts 8 teilweise in das Innere des Abschnitts 8 hineinragen, während die Hebelarme 36 schräg zu einer Längsachse B der Aufnahme 4 ausgerichtet nicht in das Innere der Aufnahme 8 hineingreifen. In dieser Stellung sind die Hebel 32 festgelegt, da die an den Hebelarmen 36 ausgebildeten Vorsprünge 42 in die an dem Griffteil 6 ausgebildeten Nuten 44 eingreifen. Hierbei sind die Schraubenfedern 24 zwischen dem Kragen 14 der Aufnahme 4 und dem Griffteil 6 gespannt.

Es besteht ein freier Zugang für das Okular 2 in das Innere des Abschnitts 8 der Aufnahme 4. Wird das Okular, wie in Fig. 4 dargestellt, in das Innere des Abschnitts 8 der Aufnahme 4 eingeführt, kontaktiert das Okular 2, bevor es an dem Kragen 12 des Abschnitts 8 zur Anlage kommt, die teilweise in das Innere des Abschnitts 8 ragenden Teile der Hebelarme 34 und drückt die Hebel 32 gegen die Federkraft der Federbügel 38 in eine Stellung, in der sich das Griffteil 6 von den Hebelarmen 36 löst und die Hebelarm 36 derart in Richtung der Längsachse B der Aufnahme 4 verschwenkt werden, dass die an dem Hebelarmen 36 ausgebildeten Vorsprünge 40 an einem konisch abgeschrägten Bereich des Okulartrichters 10 des Okulars 2 zur Anlage kommen und so den proximalen Endbereich des Okulars 2 formschlüssig umgreifen. Insofern bilden die Hebelarme 34 Steuerelemente für die Bewegung der Hebelarme 36, die wiederum Befestigungselemente zum Festlegen des Okulars 2 in der Aufnahme 4 bilden.

Die Bewegung der Hebelarme 36 der Hebel 32 in Richtung des Inneren des Abschnitts 8 hat außerdem zur Folge, dass die Vorsprünge 42 der Hebelarme 36 an den an dem Griffteil 6 ausgebildeten Nuten 44 außer Eingriff treten. Hierdurch wird das Griffteil 6 relativ zu der Aufnahme 4 durch Entspannung der Schraubenfeder 24 selbsttätig in eine Stellung bewegt, in der es direkt außenseitig der Vorsprünge 42 der Hebelarme 36 angeordnet ist und so die Hebelarme 36 an einer Bewegung von dem Okular 2 weg hindert und auf diese Weise die Hebelarme 36 in ihrer das Okular 2 festlegenden Stellung verriegelt.

Zum Lösen des Okulars 2 in der Aufnahme 4 wird das Griffteil 6 in Einführrichtung des Okulars 2 in die Aufnahme 4 gegen die Federkraft der Schraubenfedern 24 verschoben. Hierdurch wird der Kontakt der Vorsprünge 42 der Hebelarme 36 mit der Innenseite des Griffteils 6 aufgehoben, wodurch die Hebel 32 aufgrund der vorgespannten Federbügel 38 so verschwenkt werden, dass sich die Hebelarme 36 von dem Okular 2 weg nach außen bewegen und das Okular 2 zur Entnahme aus der Aufnahme 4 freigeben. Gleichzeitig greifen die an den Hebelarmen 36 ausgebildeten äußeren Vorsprünge 42 wieder in die an dem Griffteil 6 ausgebildeten Nuten 44 ein und legen das Griffteil 6 in dieser Stellung bei gespannter Schraubenfeder 24 fest.

### Bezugszeichenliste

- 2 -: Okular
- 4 -: Aufnahme
- 6 -: Griffteil
- 8 -: Abschnitt
- 10 -: Okulartrichter
- 12 -: Kragen
- 14 -: Kragen
- 16 -: Öffnung
- 18 -: Abschnitt
- 20 -: Kragen
- 22 -: Erweiterung
- 24 -: Schraubenfeder
- 26 -: Nut
- 28 -: Sicherungsring
- 30 -: Durchbrechung
- 32 -: Hebel
- 34 -: Hebelarm
- 36 -: Hebelarm
- 38 -: Federbügel
- 40 -: Vorsprung
- 42 -: Vorsprung
- 44 -: Nut
- A -: Schwenkachse
- B -: Längsachse

## Patentansprüche

1. Kupplungsvorrichtung zum lösbaren Verbinden eines Okulars (2) einer Endoskop-Optik mit einem Kameraobjektiv, mit einer Aufnahme (4) für das Okular (2), mit Befestigungsmitteln zur Befestigung des Okulars (2) in der Aufnahme (4), die durch das Okular (2) derart gesteuert sind, dass beim Einführen des Okulars (2) in die Aufnahme (4) eine selbsttätige Befestigung des Okulars (2) in der Aufnahme (4) erfolgt, und mit einem außenseitig angeordneten, manuell betätigbaren Griffteil (6) zum Lösen der Befestigung, welches nur zum Lösen gegen Federkraft in Einführrichtung des Okulars (2) in die Aufnahme (4) manuell verschiebbar ist, wobei in der Aufnahme (4) mindestens ein federvorgespanntes Steuerelement bewegbar angeordnet ist, welches mit einem Befestigungselement (36) zur Befestigung des Okulars (2) in der Aufnahme (4) bewegungsgekoppelt ist, **dadurch gekennzeichnet, dass** ein Bauteil (32) sowohl das Steuerelement (34) als auch das Befestigungselement (36) bildet, dass das Befestigungselement (36) in einer das Okular (2) in der Aufnahme (4) festlegenden Stellung von dem Griffteil (6) verriegelt ist und dass das Griffteil (6) in einer das Okular (2) freigebenden Stellung mit dem Befestigungselement festlegbar ist.

2. Kupplungsvorrichtung nach Anspruch 1, **dadurch gekennzeichnet, dass** mindestens ein abgewinkelter Hebel (32) mit zwei Hebelarmen (34, 36) vorgesehen ist, welcher um eine im Schnittbereich der beiden Hebelarme angeordnete Achse (A) schwenkbar ist, wobei ein erster in die Aufnahme (4) hineinragender Hebelarm (34) das Steuerelement bildet und ein im Wesentlichen senkrecht zu dem ersten Hebelarm (34) ausgerichteter zweiter Hebelarm (36) das Befestigungselement bildet,

3. Kupplungsvorrichtung nach Anspruch 2, **dadurch gekennzeichnet, dass** der Hebel (32) mittels eines Federbügels (38) vorgespannt ist.

4. Kupplungsvorrichtung nach einem der Ansprüche 2 oder 3, **dadurch gekennzeichnet, dass** an dem das Befestigungselement bildenden Hebelarm (36) ein Vorsprung (40) ausgebildet ist, welcher einen Okulartrichter (10) des Okulars (2) in einer Befestigungsstellung formschlüssig umgreift.

5. Kupplungsvorrichtung nach einem der Ansprüche 2 bis 4, **dadurch gekennzeichnet, dass** an dem das Befestigungselement bildenden Hebelarm (36) an der von dem Vorsprung (40) abgewandten Seite ein zweiter Vorsprung (42) ausgebildet ist.

6. Kupplungsvorrichtung nach Anspruch 5, **dadurch gekennzeichnet, dass** der zweite Vorsprung (42) des das Befestigungselement bildenden zweiten Hebelarms (36) zum Eingriff in eine an dem Griffteil (6) ausgebildete Ausnehmung (44) vorgesehen ist.

7. Kupplungsvorrichtung nach einem der Ansprüche 2 bis 6, **dadurch gekennzeichnet, dass** an einem die Aufnahme (4) bildenden Abschnitt (8) drei voneinander beabstandete Hebel (32) angeordnet sind.

## Claims

1. A coupling device for releasably connecting an eyepiece (2) of endoscope optics to a camera objective, with a receiver (4) for the eyepiece (2), with fastening means for fastening the eyepiece (2) in the receiver (4), said fastening means being controlled by the eyepiece (2) in a manner such that an automatic fastening of the eyepiece (2) in the receiver (4) is effected on introducing the eyepiece (2) into the receiver (4), and with a manually actuatable grip part (6) which is arranged at the outer side for releasing the fastening, said grip part being manually displaceable in the insertion direction of the eyepiece (2) into the receiver (4) against spring force only for release, wherein at least one spring-biased control element is movably arranged in the receiver (4), said control element being coupled in movement to a fastening element (36) for fastening the eyepiece (2) in the receiver (4), **characterised in that** one component (32) forms the control element (34) as well as the fastening element (36), that the fastening element (36) is locked in a position which fixes the eyepiece (6) in the receiver (4), by way of the grip part (6) and that the grip part (6) can be fixed in a position which releases the eyepiece (2), by way of the fastening element.

2. A coupling device according to claim 1, **characterised in that** at least one angled lever (32) with two lever arms (34, 36) is provided, said lever being pivotable about an axis (A) which is arranged in the intersection region of the two lever arms, wherein a first lever arm (34) which projects into the receiver (4) forms the control element and a second lever arm (36) which is aligned essentially perpendicularly to the first lever arm (34) forms the fastening element.

3. A coupling device according to claim 2, **characterised in that** the lever (32) is biased by way of a spring bow (38).

4. A coupling device according to one of the claims 2 or 3, **characterised in that** a projection (40) which positively engages around an eyepiece funnel (10) of the eyepiece (2) in a fastening position is formed on the lever arm (36) which forms the fastening element.

5. A coupling device according to one of the claims 2 to 4, **characterised in that** a second projection (42) is formed on the lever arm (36) which forms the fastening element, on the side which is away from the projection (40).

6. A coupling device according to claim 5, **characterised in that** the second projection (42) of the second lever arm (36) which forms the fastening element is provided for engagement into a recess (44) which is formed on the grip part (6).

7. A coupling device according to one of the claims 2 to 6, **characterised in that** three levers (32) which are distanced to one another are arranged on a section (8) which forms the receiver (4).

## Revendications

1. Dispositif de couplage pour une connexion amovible entre un oculaire (2) d'une optique d'endoscope et l'objectif d'une caméra, avec un logement (4) pour l'oculaire (2), avec des moyens de fixation pour fixer l'oculaire (2) dans le logement (4) qui sont commandés par l'oculaire (2) de façon que, lors de l'insertion de l'oculaire (2) dans le logement (4), une fixation automatique de l'oculaire (2) dans le logement (4) soit effectuée, et avec une partie poignée (6) disposée à l'extérieur et adaptée pour pouvoir être actionnée manuellement, pour détacher la fixation, laquelle partie ne peut être déplacée manuellement en coulissement, dans la direction d'insertion de l'oculaire (2) dans le logement (4), à l'encontre d'un effort de ressort, que pour le détachement, dans le logement (4), au moins un élément de commande précontraint par un ressort étant disposé mobile, qui est couplé en mouvement avec un élément de fixation (36) pour la fixation de l'oculaire (2) dans le logement (4), **caractérisé en ce qu'**un composant (32) constitue aussi bien l'élément de commande (34) que l'élément de fixation (36), **en ce que**, dans une position immobilisant l'oculaire (2) dans le logement (4), l'élément de fixation (36) est verrouillé par la partie poignée (6) et **en ce que**, dans une position détachant l'oculaire (2), la partie poignée (6) est susceptible d'être immobilise avec l'élément de fixation.

2. Dispositif de couplage selon la revendication 1, **caractérisé en ce qu'**au moins un levier coudé (32) ayant deux bras de levier (34, 36) est prévu qui est pivotant autour d'un axe (A) disposé dans la zone de coupe des deux bras de levier, un premier bras de levier (34) s'étendant jusque dans le logement (4) constituant un élément de commande et un deuxième bras de levier (36) orienté sensiblement perpendiculairement au premier bras de levier (34) constituant l'élément de fixation.

3. Dispositif de couplage selon la revendication 2, **caractérisé en ce que** le levier (32) est précontraint au moyen d'une bride de ressort (38).

4. Dispositif de couplage selon l'une des revendications 2 ou 3, **caractérisé en ce qu'**une saillie (40) est formée au bras de levier (36) constituant l'élément de fixation, laquelle entoure, dans une position de fixation, un cône d'oculaire (10) de l'oculaire (2) par complémentarité de forme.

5. Dispositif de couplage selon l'une des revendications 2 à 4, **caractérisé en ce qu'**une deuxième saillie (42) est formée au bras de levier (36) constituant l'élément de fixation, au côté opposé à la saillie (40).

6. Dispositif de couplage selon la revendication 5, **caractérisé en ce que** la deuxième saillie (42) du deuxième bras de levier (36) constituant l'élément de fixation est prévue pour l'engagement dans un évidement (44) formé à la partie poignée (6).

7. Dispositif de couplage selon l'une des revendications 2 à 6, **caractérisé en ce que** trois leviers (32) éloignés les uns des autres sont disposés dans une section (8) formant le logement (4).
